# EUROPEAN PATENT APPLICATION

(11) **EP 4 729 630 A1**
(43) Date of publication of application: **22.04.2026**
(21) Application number: 24216429.1
(22) Date of filing: 29.11.2024
(51) Int. Cl.: C12Q 1/6839

(54) **MARKER AND METHOD FOR ANALYSING A BIOLOGICAL SAMPLE**

(30) Priority: 15.10.2024 EP 24206660; 15.10.2024 EP 24206662
(71) Applicant: Leica Microsystems CMS GmbH, 35578 Wetzlar (DE)
(72) Inventor: Bradl, Joachim, 35578 Wetzlar (DE); Alsheimer, Soeren, 35578 Wetzlar (DE)
(74) Representative: Schaumburg und Partner Patentanwälte mbB

(57) **Abstract**

In a first aspect, a marker (100, 300, 400) for analysing biological sample is provided. The marker (100, 300, 400) comprises an affinity reagent (108, 406, 408) configured to specifically bind to a target analyte (110, 500) of the biological sample. The marker (100, 300, 400) further comprises a labelling moiety (106, 200, 414, 416), and a nucleic acid construct (102, 302) configured to associate the labelling moiety (106, 200, 414, 416) with the affinity reagent (108, 406, 408). The nucleic acid construct (102, 302) comprises a triplex structure (104). In a further aspect, a method for analysing the biological sample is provided.

## Description

### Technical field

The invention relates to a marker for analysing a biological sample, the marker comprising a nucleic acid construct with a triplex structure. In another aspect, a system for marking a target analyte of a biological sample and a method for analysing a biological sample with the marker is provided.

### Background

Labels and markers are frequently used when analysing biological samples, for example in fluorescent microscopy. Markers for fluorescent microscopy generally comprise affinity reagents, such as antibodies, and labels, such as fluorescent dyes, attached to the affinity reagents, in order to enable specifically attaching the labels-in particular via the affinity reagents - to a target analyte in a biological sample. This allows the identification and/or localisation of the target analyte in the biological sample.

The various applications of markers include multiplexing approaches, which require large sets of distinguishable markers, whilst in particular detecting analytes that only occur in small quantities requires markers with bright labels. It is of constant interest to provide markers for microscopy applications that allow flexible use and high reliability when detecting and identifying target analytes.

### Summary

It is an object to provide reliable markers and methods for analysing biological samples that are flexible to use and easy to assemble.

The aforementioned object is achieved by the subject-matter of the independent claims. Advantageous embodiments are defined in the dependent claims and the following description.

In a first aspect, a marker for analysing biological sample is provided. The marker comprises an (first) affinity reagent configured to specifically bind to a target analyte of the biological sample. The marker further comprises a (first) labelling moiety, and a (first) nucleic acid construct configured to associate, in particular binding or attaching, the labelling moiety with the affinity reagent. The nucleic acid construct comprises a triplex structure.

The features of the marker enable easily generating or assembling the marker from its individual parts whilst allowing flexibility in how to attach the individual parts to each other, for example based on the complementary nucleic acid strands of the nucleic acid construct. In particular, providing the nucleic acid construct with a triplex structure enables using the specificity of three nucleic acid strands hybridising to each other to bind the labelling moiety to the affinity reagent. Additionally, the triplex structure provides a particular robust and rigid association of the labelling moiety with the affinity reagent.

Importantly, using a duplex structure or dsDNA for barcoding affinity reagents, in particular antibodies, may be advantageous also in that in a dsDNA the nucleobases are hybridized to each other. Therefore, a dsDNA barcode is less likely to interact with the antibody, or more generally the barcoded affinity reagent, than a ssDNA barcode, as such interactions are mostly mediated by the nucleobases and not by the backbone of the barcode. For this reason, dsDNA barcodes are less likely to interfere with the function of the antibody and conversely the antibody-barcode interactions are less likely to interfere with the function of the barcode. Similarly, the dsDNA has a longer persistence length and therefore the label, that is attached to it has a higher distance to the antibody, which reduces label-antibody interactions which may impair antibody function, i.e. antibody binding by reducing affinity and/or specificity.

A triplex structure is a segment or section of the nucleic acid construct that comprises three single stranded nucleic acid strands, which are hybridised to each other to form a triple helix. The three single stranded nucleic acid strands do not necessarily have to be separate, individual nucleic acid molecules, rather at least some of the three single stranded nucleic acid strands may be a continuous nucleic acid molecule that hybridises to itself.

The affinity reagent may be an antibody, an antibody fragment, or an aptamer, in particular a nucleic acid or amino acid based aptamer. For example, the target analyte of the biological sample may be a protein of the biological sample and the affinity reagent may be an antibody configured to specifically bind to the target analyte.

The (first) labelling moiety may preferably be optically detectable. For example, the labelling moiety may be a fluorophore, such as a fluorescent protein, an organic or inorganic fluorescent molecule, or a fluorescent nanoparticle. Thus, the labelling moiety may be optically detectable with a fluorescence microscope. In particular, the marker may comprise several labelling moieties, in particular several of the first labelling moiety. The labelling moiety is preferably (covalently) attached to a nucleic acid strand of the nucleic acid construct.

Preferably, the nucleic acid construct comprises a first nucleic acid strand and a second nucleic acid strand configured to form a duplex structure with each other, and a third nucleic acid strand configured to form the triplex structure with the first nucleic acid strand and the second nucleic acid strand.

In particular, the first nucleic acid strand comprises a first nucleotide sequence complementary to a second nucleotide sequence of the second nucleic acid strand.

Preferably, the first nucleotide sequence and the second nucleotide sequence are at least partially complementary to each other, in particular based on Watson-Crick base pairing. Thus, the first and the second sequence may form the duplex structure with each other. This enables stable hybridisation of the first nucleotide sequence and the second nucleotide sequence to each other. The duplex may be a double helix, such as B-form DNA.

The third nucleic acid strand has a third nucleotide sequence, in particular. The third nucleotide sequence may be at least partially complementary to the hybridised first nucleotide sequence and second nucleotide sequence, in particular based on Hoogsteen base-pairing, forming the triplex structure. Thus, the third nucleotide sequence can hybridise to the duplex of the first nucleotide sequence and the second nucleotide sequence, in particular to a major groove of the duplex. This enables a stable triplex.

In particular, at least one of the first nucleic acid strand and the second nucleic acid strand is (covalently) attached to the affinity reagent.

Preferably, the first nucleic acid strand and the second nucleic acid strand are resistant to a degradation agent, and the third nucleic acid strand is sensitive to the degradation agent. This enables selectively degrading the sensitive (third) nucleic acid strand by means of the degradation agent. Preferably, the affinity reagent is similarly resistant to the degradation agent. The degradation agent may be a nuclease or a physical parameter or stimulus such as light or temperature, for example.

Preferably, the first nucleic acid strand and the second nucleic acid strand may comprise or essentially consist of nucleic acid analogues, in particular modified nucleobases. Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these parts altered. Thus, a nucleic acid analogue may be a non-naturally occurring, modified, artificial, or xeno nucleic acid (XNA), in particular. Whereas natural nucleic acids or naturally occurring nucleic acids (DNA and RNA) are generally sensitive to natural degradation agents such as nucleases, nucleic acid analogues are generally resistant to these degradation agents such as nucleases. Thus, this enables providing a marker (part), comprising the affinity reagent to which the first nucleic acid strand and the second nucleic acid strand are connected, being resistant to degradation by natural degradation agents, such as nucleases, and therefore a particular robust marker (part).

The first nucleic acid strand and the second nucleic acid strand may exhibit differential sensitivity to nucleases or other DNA-modifying enzymes. This could be used to control the formation of the duplex and thereby the ability of marker to form.

More specifically, nucleic acid analogues may comprise modified nucleobases or may comprise partially or entirely a xeno nucleic acid (XNA) like for example PT-DNA, L-DNA, LNA, morpholino, peptide nucleic acid (PNA). The use of XNA may help to improve nuclease-resistance, which is desired in some applications.

Alternatively, the first nucleic acid strand and the second nucleic acid strand may comprise or essentially consists of natural occurring nucleic acids.

Preferably, the labelling moiety is (covalently) attached to the third nucleic acid strand. This enables binding the labelling moiety to the affinity reagent with high specificity, since the presence of three complementary nucleic acid strands is required.

In a particular embodiment, the marker may have several third nucleic acid strands, which are hybridised to different parts of a duplex formed between the first nucleic acid strand and the second nucleic acid strand. Preferably, each third nucleic acid strand comprises one of the labelling moieties. This enables generating a bright marker. Alternatively, each third nucleic acid strand may comprise a labelling moiety with different, in particular distinguishable, optical properties such as excitation wavelength, emission wavelength, and emission lifetime. This enables generating a diverse set of markers with each marker having different optical properties, and each marker being distinguishable from other markers of the set of markers.

Preferably, the marker comprises a second nucleic acid construct configured to associate a second labelling moiety of the marker with the affinity reagent. The second nucleic acid construct and the second labelling moiety may have same features as (first) nucleic acid construct, in particular as described above. The second nucleic acid construct enables associating additional labelling moieties with the affinity reagent.

Preferably, the (first) labelling moiety and the second labelling moiety are configured to form an energy transfer pair. For example, the first labelling moiety may be a donor, and the second labelling moiety may be an acceptor. Specifically, the first and second labelling moieties may form a Förster resonance energy transfer (FRET) pair. This enables determining whether both, the first and second labelling moieties are in proximity and bound to the marker.

Preferably, the first nucleic acid construct and/or the second nucleic acid construct, in particular respective first and/or second nucleic acid strands, may be attached to the affinity reagent by means of a secondary affinity reagent, such as an antibody fragment. The secondary affinity reagent may be configured to specifically bind to the affinity reagent, in particular, to a part of the affinity reagent that is not configured to bind to the target analyte.

In a particular example, in case of the affinity reagent being an antibody, the secondary affinity reagent may be configured to specifically bind to a Fc region of the affinity reagent. This enables providing a set of markers with each marker having an affinity reagent of the same isotype, and each marker being specific to a different target analyte of the biological sample. The markers of the set of markers are easily assembled by bringing the affinity reagents of the same isotype being specific to the different target analytes together with nucleic acid constructs attached to secondary affinity reagents configured to bind to the Fc region of the isotype of the affinity reagents. This avoids having to covalently attach the nucleic acid constructs to the affinity reagents. Preferably, the nucleic acid constructs have labelling moieties with different optical properties. Thus, by mixing and matching affinity reagents with nucleic acid constructs, the set of markers may be efficiently generated.

In a particular embodiment, the first nucleic acid construct and the second nucleic acid construct may comprise labelling moieties with different, in particular distinguishable, optical properties such as excitation wavelength, emission wavelength, and emission lifetime. This enables generating a diverse set of markers with each marker having different optical properties, and each marker being distinguishable from other markers of the set of markers.

Preferably, the marker comprises a further affinity reagent configured to specifically bind to a target analyte of the biological sample, a further labelling moiety, in particular a plurality of the further labelling moiety, a further nucleic acid construct configured to associate the further labelling moiety with the further affinity reagent. The further nucleic acid construct comprises a triplex structure, and the marker further comprises a linking nucleic acid strand configured to form the triplex structure of the (first) nucleic acid construct and of the further nucleic acid construct. Thus, the linking nucleic acid strand is complementary to both of the nucleic acid constructs. In particular, a first linking sequence of the linking nucleic acid strand is complementary to the duplex of the first and second nucleic acid strand of the first nucleic acid construct and a further linking sequence of the linking nucleic acid strand is complementary to the duplex of the first and second nucleic acid strand of the further nucleic acid construct. The hybridisation of the linking nucleic acid strand forms the triplex structure of the respective nucleic acid construct. The hybridisation of the linking nucleic acid strand to the respective nucleic acid construct is preferably based on Hoogsteen base-pairing. In particular, the first affinity reagent, the first nucleic acid construct and the first labelling moiety may be part of a first marker part of the marker. In contrast, the further affinity reagent, the further nucleic acid construct and the further labelling moiety may be part of a further marker part of the marker. Providing the linking nucleic acid strand enables linking both marker parts together, in particular, when they are in close proximity. The first affinity reagent and the further affinity reagent may be configured to specifically bind to the same or to different target analytes. When the first affinity reagent and the further affinity reagent are configured to specifically bind to the same target analyte, they may bind to different regions or epitopes of the same target analyte.

The first labelling moiety and the further labelling moiety are preferably covalently attached to the first and/or second strand of the respective nucleic acid construct. The marker may have pluralities of the first and/or further labelling moiety.

The third nucleic acid strand and/or the linking nucleic acid strand is preferably a naturally occurring nucleic acid. For example, this enables selectively degrading the third nucleic acid strand and/or the linking nucleic acid strand in case the first and second nucleic acid strands are nucleic acid analogues.

Preferably, the (first) labelling moiety and the further labelling moiety are configured to form an energy transfer pair. Specifically, the first and further labelling moieties may form a Förster resonance energy transfer (FRET) donor/acceptor pair. This enables determining whether both, the first and second labelling moieties and therefore the first marker part and the further marker part are in proximity.

The marker comprising the further affinity reagent, the further labelling moiety, and the further nucleic acid construct may be used in a proximity assay, for example. In this case, the first marker part and the further marker part may bind to different target analytes. In case the different target analytes are in proximity the linking nucleic acid strand may hybridise to both, the first and the further nucleic acid constructs, to form their respective triplex structures and bind the marker parts together. In particular, this brings the respective nucleic acid constructs with the labelling moieties in proximity. This proximity can be detected e.g. based on an optical readout (in particular with a fluorescence microscope) when the first labelling moiety and the further labelling moiety are an energy transfer pair, for example, by a change of an emission wavelength of the first labelling moiety and the further labelling moiety pair.

In a further aspect, a system for (iteratively) marking a target analyte of a biological sample is provided. The system comprises at least one marker according, in particular according to the above description. The system further comprises a plurality of labelling moieties, with each labelling moiety attached to one of a plurality of the third nucleic acid strand. The plurality of labelling moieties each have different, in particular distinguishable, optical properties. Thus, the different labelling moieties may each be associated with the affinity reagent by hybridisation of the respective third nucleic acid strand to the nucleic acid construct of the affinity reagent. In particular, several labelling moieties may be attached to each one of the plurality of third nucleic acid strands. Preferably, the combination of the several labelling moieties of each third nucleic acid strand is different for, in particular unique to, each third nucleic acid strand.

The system may be used for (iteratively) marking target analytes of a biological sample. For example, by iteratively hybridising and subsequently removing each third nucleic acid strand, a particular target analyte may be marked with a unique combination of labelling moieties and identified and/or localised in the biological sample based on that unique combination, e.g. based on subsequent (iterative) optical readouts of the marked biological sample.

The system may comprise several markers. Each marker of the system may have a plurality of labelling moieties, with each labelling moiety attached to one of a plurality of the third nucleic acid strand, which are configured to hybridise specifically to one of the markers, in particular their respective nucleic acid constructs. Preferably, the plurality of labelling moieties for each marker may have different, in particular distinguishable, optical properties.

In another aspect, a method for analysing a biological sample is provided. The method comprises the following steps: Introducing at least one marker, in particular according to the above description, into the biological sample, and generating a readout of the biological sample with the at least one marker.

During the step of introducing the at least one marker, elements of the marker, such as the affinity reagent, and the third nucleic acid strand with the labelling moiety may be introduced separately from and/or subsequently to each other. The marker may then be generated in the biological sample. Alternatively, the marker may be generated prior to introducing it into the biological sample.

Thus, the marker may either be generated prior to introduction into the biological sample or the marker may be generated from its parts within the biological sample. The latter may be advantageous in case the marker, in particular the label, is bulky and/or rigid compared to its individual parts and the biological sample is a tissue section, for example. In this case, the more compact and/or flexible individual parts may be introduced separately and the bulky and/or rigid marker is only generated in situ. In particular, the formation of the triplex structure of the nucleic acid construct in situ may be advantageous, since the individual nucleic acid strands may more easily penetrate or diffuse into the biological sample compared to the duplex/triplex structure. Thus, preferably the duplex/triplex structures of the marker may be generated in situ, for example by adding the third nucleic acid strand after adding the affinity reagent with the first and second nucleic acid strands.

The readout may be an optical readout, for example, generated by means of an optical device such as a microscope or an imaging spectrometer. Target analytes to which the marker binds to may be identified and/or localised in the readout.

Preferably, the method comprises a step of applying a degradation agent to the biological sample, in particular, after introducing marker the marker and/or after generating the readout. In particular, this may degrade the third nucleic acid strand sensitive to the degradation agent and remove it from the marker. This enables binding of another third nucleic acid strand to the first and second nucleic acid strands of the marker. The degradation agent may be a nuclease, for example, or a physical parameter or stimulus such as a pH change, a temperature change, or applied to the sample. In a particular embodiment, the degradation agent may merely cause the dissociation of the third nucleic acid strand from the duplex of the first and second nucleic acid strands. For example, a temperature increase may cause the third nucleic acid strand to dissociate from the first and second nucleic acid strands, in particular if the temperature increase is above a respective melting temperature of the third nucleic acid strand hybridised to the first and second nucleic acid strands.

Preferably, at least one of a plurality of labelling moieties attached to respective third nucleic acid strands is introduced into the biological sample. This step is preferably carried out after applying the degradation agent. In particular, the labelling moieties have different optical properties, if several markers were introduced initially. In this case, the third nucleic acid strands may be specific to particular markers or their respective nucleic acid constructs. For example, labelling moieties attached to respective third nucleic acid strands from the system described above may be used in this step. Subsequently to this step, another readout may be generated. The steps of the method may be iteratively repeated with different labelling moieties associated with a particular affinity reagent in each step, in order to identify target analytes based on the combination of labelling moieties associated with the target analytes over the iterations.

Preferably, the at least one marker is introduced into the biological sample under a first condition, and prior to generating the optical readout, a second condition is applied to the biological sample. In this case, the at least one marker may comprise a first marker part and a further marker part, as described above, in particular.

For example, under the first condition, the duplex and/or triplex structure of the first and/or second nucleic acid constructs of the marker cannot be generated. Whereas under the second condition, the duplex and/or triplex structure of the nucleic acid construct can be generated. The first and second conditions may refer to an environmental parameter, such as a particular temperature, salt concentration, formamide concentration, or a concentration of a competitively binding nucleic acid strand. The competitively binding nucleic acid strand may be selectively digested under the second condition, for example.

As a specific example, the duplex/triplex structure of the nucleic acid construct may be melted by an increase in temperature as the first condition, in particular above the melting temperature of the duplex/triplex structure. This causes the nucleic acid strands of the construct to relax or disassociate. The duplex/triplex structure may be generated by subsequently reducing the temperature as the second condition, causing the nucleic acid construct to form, in particular the duplex/triplex structure. Thus, applying different conditions to the biological sample when introducing the marker into the biological sample and when generating the readout enables efficiently introducing the marker.

Generally, a plurality of markers may be introduced into the biological sample, the markers being specific to respective target analytes, in order to identify a large number of (different or the same) target analytes at the same time. Preferably, a target analyte is identified and/or localised within the biological sample based on the label(s) of the marker(s), in particular the labelling moieties, associated with the target analyte in the optical readout.

The system and the method have the same advantages as the marker. Further, the system and the method may be supplemented with the features of the marker described in this document, in particular, the features of the dependent claims of the marker.

### Short Description of the Figures

Hereinafter, specific embodiments are described referring to the drawings, wherein:
- Figure 1: is a schematicviewof a marker comprising a nucleic acid construct with a triplex structure and a labelling moiety,
- Figure 2: is a schematic view of steps to swap the labelling moiety of the marker according to Fig. 1,
- Figure 3: is a schematic view of a marker with two nucleic acid constructs,
- Figure 4: is a schematic view of a marker with a first marker part and a second marker part, and
- Figure 5: is a schematic view of the marker according to Fig. 4 with the marker parts in proximity.

### Detailed Description

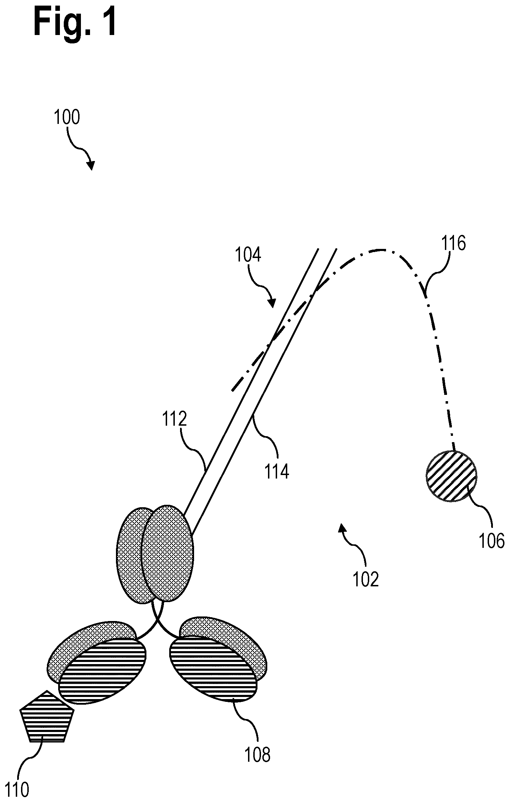

Figure 1 is a schematic view of a marker 100 comprising a nucleic acid construct 102 with a triplex structure 104 and a labelling moiety 106, such as a fluorophore. The marker 100 further comprises an affinity reagent 108, in particular an antibody, configured to specifically bind to a target analyte 110.

The nucleic acid construct 102 comprises a first nucleic acid strand 112 and a second nucleic acid strand 114, which may both be attached to the affinity reagent 108, in particularly covalently attached. The first nucleic acid strand 112 and the second nucleic acid strand 114 comprise respective nucleotide sequences that are complementary to each other. This complementarity is based on Watson-Crick base-pairing, in particular. Thus, the first nucleic acid strand 112 and the second nucleic acid strand 114 may hybridise and form a duplex structure, in particular a B-form double helix. In Fig. 1 the duplex structure is for simplicity schematically shown as two parallel lines.

The nucleic acid construct 102 further comprises a third nucleic acid strand 116. The third nucleic acid strand 116 comprises a third nucleotide sequence that is complementary to at least a part of the duplex structure of the first and second nucleic acid strands 112, 114. This complementarity is based on Hoogsteen base-pairing, in particular. Thus, the third nucleic acid strand 116 may hybridise to at least the part of the duplex structure and form the triplex structure 104 with the first and second nucleic acid strands 112, 114. In particular, the third nucleic acid strand 116 may hybridise to the major groove of the duplex structure.

The triplex structure 104 of the nucleic acid construct 102 of the marker 100 enables binding the labelling moiety 106 to the affinity reagent 108 simply by mixing the third nucleic acid strand 116 having the labelling moiety 106 with the affinity reagent 108 having the first and second nucleic acid strands 112, 114. Performing a chemical reaction is not required. However, the complementarity between the third nucleic acid strand 116 and the first and second nucleic acid strands 112, 114 still ensures specific association of the labelling moiety 106 with the affinity reagent 108.

Optionally, several of the labelling moieties 106 may be attached to the third nucleic acid strand 116. These several labelling moieties 106 may all have the same optical properties to enable a bright marker. Alternatively, these several labelling moieties 106 may have different optical properties to enable generating a large number of markers 100 that can be distinguished by their unique combination of labelling moieties 106 with different optical properties.

As a further option, the marker 100 may comprise several of the third nucleic acid strand 116. For example, these several third nucleic acid strands 116 may each comprise different nucleotide sequences that are complementary to different parts of the duplex structure of the first and second nucleic acid strands 112, 114. This enables attaching many labelling moieties 106 to the affinity reagent 108.

By introducing the marker 100 into a biological sample (not shown), the affinity reagent 108 may bind to the respective target analyte 110 in the biological sample. Subsequently, an optical readout of the biological sample may be generated, for example by means of a microscope. In the optical readout, the presence and/or location of the target analyte 110 may be determined based on detecting the optical properties of the labelling moiety 106 in the optical readout.

In particular, several different markers may be introduced into the biological sample, with each marker having an affinity reagent configured to specifically bind to a different target analyte of the biological sample, each marker with a particular affinity reagent may have a labelling moiety with the same optical properties. Thus, the different target analytes may be identified in the biological sample based on detecting the optical properties of the respective labelling moieties in the optical readout.

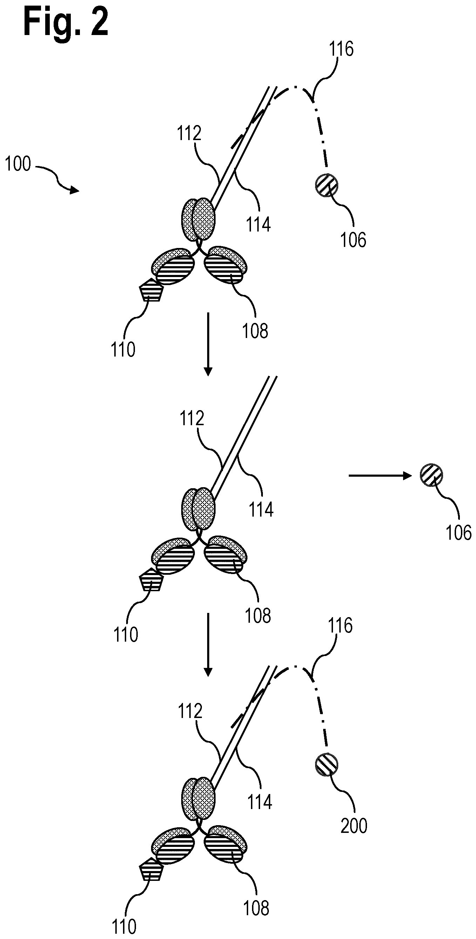

Figure 2 is a schematic view of steps to swap the labelling moiety 106 of the marker 100. Initially, the third nucleic acid strand 116 with the labelling moiety 106 is associated with the affinity reagent 108 via the nucleic acid construct 102 (see top part of Figure 2). At this point, an initial (optical) readout of the biological sample together with the marker 100 with the labelling moiety 106 attached to the target analyte 110 might be generated. Next, a degradation agent (not shown) is applied to the marker 100, causing the third nucleic acid strand 116 to dissociate from the first and second nucleic acid strands 112, 114 (see middle part of Figure 2). In particular, the third nucleic acid strand 116 may be degraded, for example in case the degradation agent is a nuclease, the third nucleic acid strand 116 may be digested (in particular, if the first and second nucleic acid strands 112, 114 are resistant to the degradation agent). This in turn dissociates the labelling moiety 106 from the affinity reagent 108.

In a particular embodiment, the first and second nucleic acid strands 112, 114 of the marker 100 may be resistant to the degradation agent and the third nucleic acid strand 116 may be sensitive to the degradation agent to enable selectively degrading the third nucleic acid strand 116. To that end, the first and second nucleic acid strands 112, 114 may be nucleic acid analogues, which are resistant to a nuclease degradation agent. The third nucleic acid strand 116 may be a natural nucleic acid, which is sensitive to the nuclease degradation agent.

Alternatively, the degradation agent may be a physical parameter such as a temperature. An increase of the temperature the marker 100 is at, may cause the third nucleic acid strand 116 to dissociate from the first and second nucleic acid strands 112, 114 and therefore cause the degradation of the association of the labelling moiety 106 with the affinity reagent 108.

In a next step after applying the degradation agent, a further third nucleic acid strand 116 with a second labelling moiety 200 is brought into contact with the first and second nucleic acid strands 112, 114. The further third nucleic acid strand 116 may then hybridise to the first and second nucleic acid strands 112, 114 to form the triplex structure 104. This associates the second labelling moiety 200 with the affinity reagent 108 (see bottom part of Figure 2). A further (optical) readout of the biological sample with the marker 100 with the second labelling moiety 200 might be generated.

The steps described above for Fig. 2 may be iteratively repeated in order to identify a large number of target analytes with a limited number of distinguishable labelling moieties.

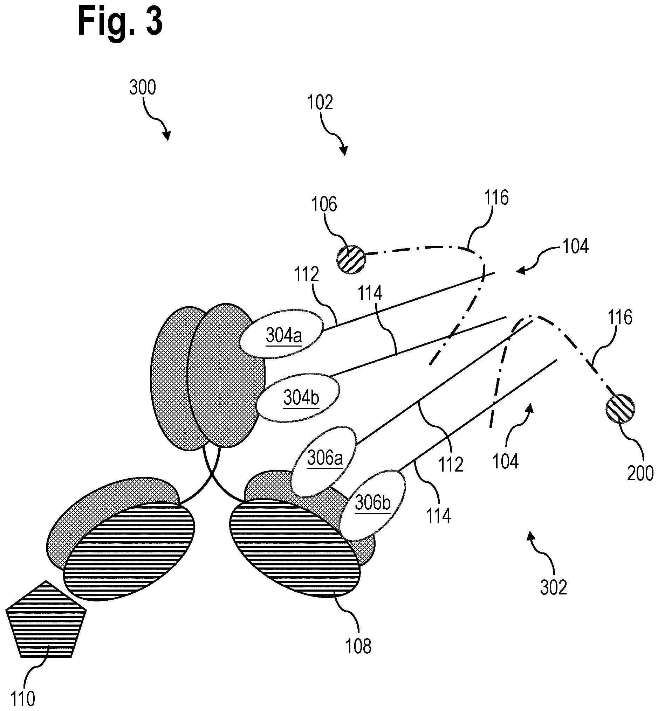

Figure 3 is a schematic view of a marker 300 with two nucleic acid constructs 102, 302. Each nucleic acid construct comprises the first, second, and third nucleic acid strand 112, 114, 116, which may form the triplex structure 104, when the third nucleic acid strand 116 binds to the first and second nucleic acid strands 112, 114.

The first nucleic acid construct 102 comprises the third nucleic acid strand 116 with the labelling moiety 106 and therefore associates the labelling moiety 106 with the affinity reagent 108. The second nucleic acid construct 302 comprises the third nucleic acid strand 116 with the second labelling moiety 200 and therefore associates the second labelling moiety 200 with the affinity reagent 108.

The nucleic acid constructs 102, 302 are bound to the affinity reagent 108 via secondary affinity reagents 304a, 304b, 306a, 306b. In particular, the first and second nucleic acid strands 112, 114 of the first nucleic acid construct 102 are bound to the affinity reagent 108 via a respective first and second secondary affinity reagents 304a, 304b. Similarly, the first and second nucleic acid strands 112, 114 of the second nucleic acid construct 302 are bound to the affinity reagent 108 via a respective first and second secondary affinity reagents 306a, 306b. Each of the first and second nucleic acid strands 112, 114 is preferably covalently attached to the respective secondary affinity reagent 304a, 304b, 306a, 306b.

The secondary affinity reagents 304a, 304b, 306a, 306b are each configured to specifically bind to the affinity reagent 108. In particular, the secondary affinity reagents 304a, 304b, 306a, 306b are each configured to specifically bind to different regions of the affinity reagent 108 such that they their binding is not competitive. For example, the secondary affinity reagents 304a, 304b, 306a, 306b may bind to heavy chains, light chains, Fab-fragment, and/or Fc-fragment of the antibody affinity reagent 108.

In a particular embodiment of the marker 300, the secondary affinity reagents 304a, 304b, 306a, 306b are configured to bind to a conserved region of the affinity reagent 108, for example, to the Fc-fragment of the affinity reagent 108. This enables generating the marker 300 by mixing the nucleic acid constructs 102, 302 attached to the secondary affinity reagents 304a, 304b, 306a, 306b with the affinity reagent 108. Due to their specific affinity, the secondary affinity reagents 304a, 304b, 306a, 306b will bind to their respective epitopes on the affinity reagent 108.

By providing a plurality of nucleic acid constructs 102, 302 having labelling moieties 106, 200 with distinguishable optical properties, as well as a plurality of affinity reagents 108 being configured to specifically bind to different target analytes, a set of the markers 300 may be generated that comprises markers 300 that are specific to different target analytes, with markers 300 specific to the same target analyte having labelling moieties with the same optical properties.

In a specific embodiment, the secondary affinity reagents 304a, 304b, 306a, 306b may be configured to specifically bind to an Fc-fragment of the antibody affinity reagent 108. Thus, when generating a set of markers 300 the Fc-fragment may be conserved across all affinity reagents 108, which may nevertheless be specific to different target analytes. The secondary affinity reagents 304a, 304b, 306a, 306b enable efficiently attaching the nucleic acid constructs 102, 302 to any one of the affinity reagents 108.

In an alternative embodiment, the marker 300 may only comprise a single nucleic acid construct 102, 302, or a different number of nucleic acid constructs 102, 302.

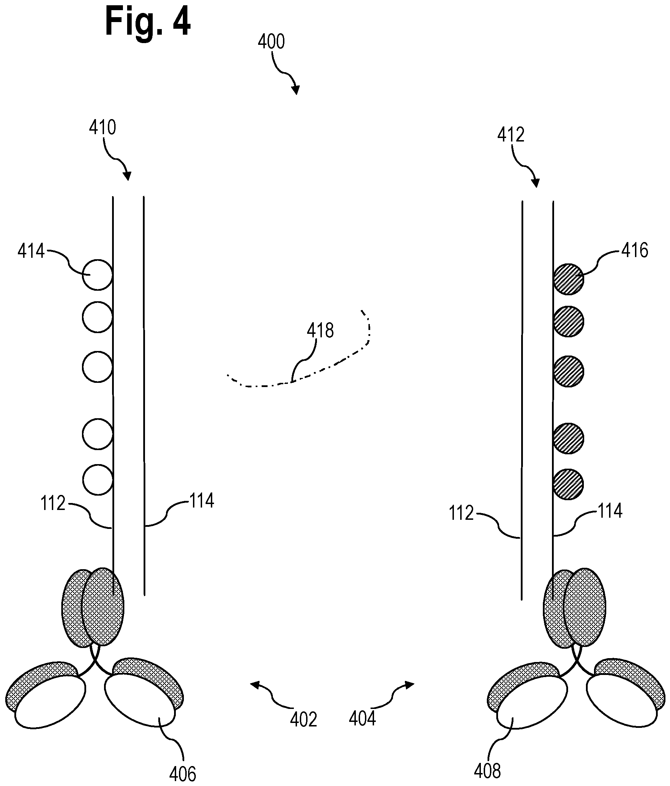

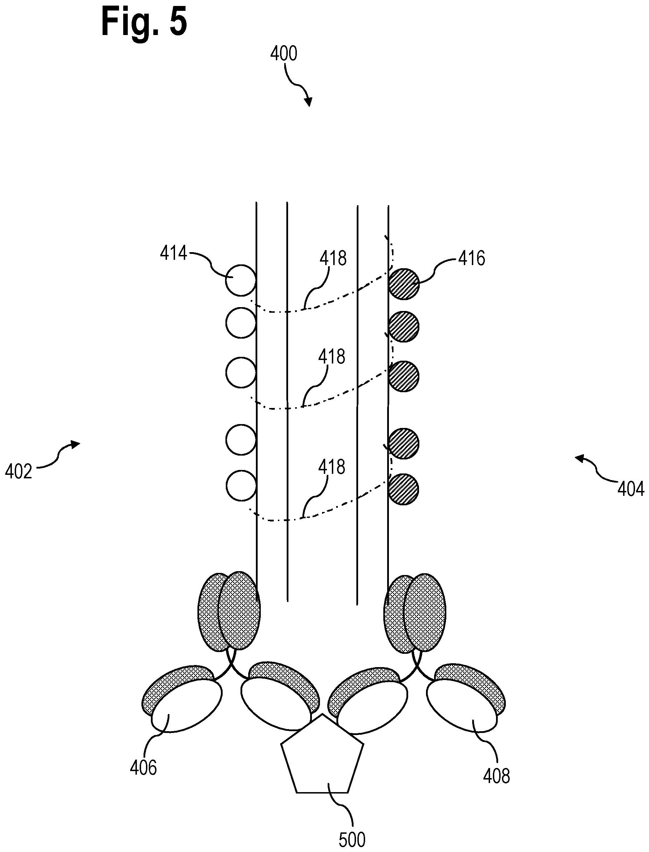

Figures 4 and 5 are schematic views of a marker 400 with a first marker part 402 and a second marker part 404. The first marker part 402 comprises a first affinity reagent 406, a nucleic acid duplex 410 of a first nucleic acid construct, and several first labelling moieties 414. Similarly, the second marker part 404 comprises a second affinity reagent 408, a nucleic acid duplex 412 of a second nucleic acid construct, and several second labelling moieties 416.

The duplexes 410, 412 each comprise the first and second nucleic acid strands 112, 114, which may hybridise to each other to form a duplex structure. Moreover, the duplexes 410, 412 may form a triplex structure with a third nucleic acid strand 418 to form the first nucleic acid construct and the second nucleic acid construct. The third nucleic acid strand 418 has a first sequence, which is complementary to at least a part of the duplex 410, and a second sequence, which is complementary to at least a part of the duplex 412. The third nucleic acid strand 418 may alternatively be called a linking nucleic acid strand. As described above, the complementarity may be based on Hoogsteen base-pairing between the third nucleic acid strand 418 and the duplexes of the first and second nucleic acid strands 112, 114 of the duplexes 410, 412. Thus, the third nucleic acid strand may hybridise to both duplexes 410, 412 and form the triplex of both, the first and the second nucleic acid construct. In Fig. 4, the first marker part 402 and the second marker part 404 are at a distance to each other, in particular the distance between the marker parts 402, 404 being greater than the length of the third nucleic acid strand 418. Further in particular, the marker parts 402, 404 are at a distance, at which the third nucleic acid strand cannot bind to both marker parts 402, 404 simultaneously. Thus, in Fig. 4 no triplex is formed.

The first and second labelling moieties 414, 416 are preferably configured for non-radiative energy transfer between them. For example, the labelling moieties 414, 416 may be a FRET pair or one of the first and second labelling moieties 414, 416 may be a quencher that quenches the fluorescent emission of the other one of the first and second labelling moieties 414, 416. Thus, when the labelling moieties 414, 416 are apart and at a distance to each other, as schematically shown in Fig. 4, no energy transfer occurs between them and the first and second labelling moieties 414, 416. In this case, the optical properties of the first and second labelling moieties 414, 416 are not influenced by the respective other of the first and second labelling moieties 414, 416.

Figure 5 is a schematic view of the marker 400 with the first marker part 402 and the second marker part 404 being arranged in close proximity to each other. In particular, the affinity reagents 406, 408 are bound to a target analyte 500. The affinity reagents 406, 408 may be antibodies, for example. The affinity reagents 406, 408 may be configured to specifically bind to the same target analyte 500 or to different target analytes. As shown in Fig. 5, the affinity reagents 406, 408 are bound to different epitopes of the same target analyte 500.

Due to the affinity reagents 406, 408 being both bound to the same target analyte 500, the marker parts 402, 404, in particular the duplexes 410, 412 of respective nucleic acid constructs, are in proximity and only then the third nucleic acid strand 418 may form a triplex with both marker parts 402, 404. Thus, the marker parts 402, 404 are bound to each other via the third nucleic acid strand 418, which keeps the marker parts 402, 404, in particular the labelling moieties 414, 416, in proximity of each other. In Fig. 5 three third nucleic acid strands 418 are shown exemplarily.

Further, due to the proximity of the marker parts 402, 404, the respective labelling moieties 414, 416 are similarly in proximity to each other, in particular within a Förster distance. This enables the non-radiative energy transfer between the labelling moieties 414, 416. This energy transfer changes the optical properties of the labelling moieties 414, 416 (in particular relative to the arrangement as shown in Figure 4), which may be observed, for example by means of a microscope.

Thus, by observing if an energy transfer is occurring between the first and second labelling moieties 414, 416, it can be determined if the marker parts 402, 404 are in proximity and therefore if the affinity reagents 406, 408 are in proximity and the target analyte 500 is present.

In case the affinity reagents 406, 408 are configured to bind to different target analytes, the observation of an energy transfer between the first and second labelling moieties 414, 416 can be used to determine if the different target analytes are bound to each other or are located in close proximity to each other.

To determine the presence of the target analyte 500 in a biological sample, the marker 400 may initially be introduced into the biological sample. Subsequently, an optical readout may be generated, for example by means of a microscope. From the optical readout, it may be determined, if an energy transfer occurs between the first and second labelling moieties 414, 416 and consequently, if the target analyte 500 is present in the biological sample.

The marker 400 may preferably be introduced into the biological sample under a first condition, under which hybridisation of nucleic acids, in particular of the third nucleic acid strand 418 to the first and second nucleic acid strands 112, 114 is not possible. For example, this may include a salt concentration, a temperature above the melting temperature of the triplex structure formed by the hybridisation of the third nucleic acid strand 418, or a competitive binding nucleic acid strand, which blocks the third nucleic acid strand 418 from binding to the first and second nucleic acid strands 112, 114. Thus, under the first condition, the triplex structure is not formed. Prior to generating the optical readout, a second condition is subsequently applied, which enables the hybridisation of the third nucleic acid strand 418 to the first and second nucleic acid strands 112, 114 and formation of the triplex structure. In case the marker parts 402, 404 are in proximity, for example due to the affinity reagents 406, 408 being bound to the target analyte 500, the third nucleic acid strand 418 may bind together the marker parts 402, 404 and an energy transfer between the labelling moieties 414, 416 may be observed in the subsequently generated optical readout.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 300, 400: Marker
- 102, 302: Nucleic acid construct
- 104: Triplex structure
- 106, 200, 414, 416: Labelling moiety
- 108: Affinity reagent
- 110, 500: Target analyte
- 112: First nucleic acid strand
- 114: Second nucleic acid strand
- 116, 418: Third nucleic acid strand
- 304a, 304b, 306a, 306b: Secondary affinity reagent
- 402: First marker part
- 404: Second marker part
- 406: First affinity reagent
- 408: Second affinity reagent
- 410, 412: Duplex of nucleic acid construct

## Claims

1. A marker (100, 300, 500) for analysing a biological sample, comprising
an affinity reagent (108, 406, 408) configured to specifically bind to a target analyte (110, 500) of the biological sample,
a labelling moiety (106, 200, 414, 416), and
a nucleic acid construct (102, 302) configured to associate the labelling moiety (106, 200, 414, 416) with the affinity reagent (108, 406, 408), and
wherein the nucleic acid construct (102, 302) comprises a triplex structure (104).

2. The marker according to claim 1, wherein the nucleic acid construct (102, 302) comprises a first nucleic acid strand (112) and a second nucleic acid strand (114) configured to form a duplex structure with each other, and a third nucleic acid strand (116, 418) configured to form the triplex structure (104) with the first nucleic acid strand (112) and the second nucleic acid strand (114).

3. The label according to claim 2, wherein the first nucleic acid strand (112) and the second nucleic acid strand (114) are resistant to a degradation agent, and wherein the third nucleic acid strand (116, 418) is sensitive to the degradation agent.

4. The marker according to one of the preceding claims 2 and 3, wherein the first nucleic acid strand (112) and the second nucleic acid strand (114) are nucleic acid analogues.

5. The marker according to one of the preceding claims 2 to 4, wherein the labelling moiety (106, 200, 414, 416) is attached to the third nucleic acid strand (116).

6. The marker according to one of the preceding claims, comprising a second nucleic acid construct (302) configured to associate a second labelling moiety (200) of the marker (300) with the affinity reagent (108).

7. The marker according to claim 6, wherein the labelling moiety (106) and the second labelling moiety (200) are configured to form an energy transfer pair.

8. The marker according to one of the preceding claims, wherein the nucleic acid construct (102, 302) is bound to the affinity reagent (108) by means of a secondary affinity reagent (304a, 304b, 306a, 306b).

9. The marker according to one of the preceding claims, comprising a further affinity reagent (408) configured to specifically bind to a target analyte (500) of the biological sample, a further labelling moiety (416), a further nucleic acid construct (412) configured to associate the further labelling moiety (416) with the further affinity reagent (408), wherein the further nucleic acid construct (412) comprises a triplex structure, the marker (400) further comprising a linking nucleic acid strand (418) configured to form the triplex structure of the nucleic acid construct and of the further nucleic acid construct.

10. The marker according to claim 9, wherein the labelling moiety (414) and the further labelling moiety (416) are configured to form an energy transfer pair.

11. A system for marking a target analyte of a biological sample comprising:
the marker (100, 300) according to one of the preceding claims 2 to 5, and
a plurality of labelling moieties (106, 200), wherein each labelling moiety (106, 200) is attached to one of a plurality of the third nucleic acid strand (116), and
wherein the plurality of labelling moieties (106, 200) each have different optical properties.

12. A method for analysing a biological sample comprising the following steps:
introducing at least one marker (100, 300, 400) according to one of the preceding claims 1 to 10 into the biological sample, and
generating a readout of the biological sample with the at least one marker (100, 300, 400).

13. The method according to claim 12, wherein a degradation agent is applied to the biological sample.

14. The method according to claim 13, wherein at least one of a plurality of labelling moieties (106, 200) attached to respective third nucleic acid strands (116) is introduced into the biological sample.

15. The method according to one of the preceding claims 12 to 14, wherein the at least one marker (100, 300, 400) is introduced into the biological sample under a first condition, and prior to generating the readout, a second condition is applied to the biological sample.
